# EUROPEAN PATENT SPECIFICATION

(11) **EP 3 405 584 B1**
(45) Date of publication and mention of the grant of the patent: **04.11.2020**
(21) Application number: 16784588.2
(22) Date of filing: 03.10.2016
(51) Int. Cl.: G01N 33/50, G01N 33/48, C12Q 1/14

(54) **CULTURE MEDIUM FOR DETECTING ENTEROCOCCUS**
KULTURMEDIUM ZUM NACHWEIS VON ENTEROKOKKEN
MILIEU DE CULTURE POUR LA DÉTECTION D'ENTÉROCOQUES

(30) Priority: 18.01.2016 JP 2016006892
(43) Date of publication of application: 28.11.2018
(73) Proprietor: JNC Corporation, Chiyoda-ku Tokyo 100-8105 (JP)
(72) Inventor: TERAMURA, Hajime, Yokohama-shi Kanagawa 2368605 (JP); IWASAKI, Mihoko, Yokohama-shi Kanagawa 2368605 (JP)
(74) Representative: Becker & Kurig Partnerschaft Patentanwälte PartmbB
(86) International application number: PCT/JP2016/004454
(87) International publication number: WO 2017/125970

(56) References cited:
- WO-A1-01/14583
- WO-A1-96/06182
- JP-A- 2008 017 712
- US-A- 5 723 308
- US-A1- 2007 026 482
- US-A1- 2009 280 524
- KONRAD J DOMIG ET AL: "Methods used for the isolation, enumeration, characterisation and identification of Enterococcus spp. 1. Media for isolation and enumeration", INTERNATIONAL JOURNAL OF FOOD MICROBIOLOGY, ELSEVIER BV, NL, vol. 88, no. 2-3, 1 December 2003 (2003-12-01), pages 147-164, XP002567434, ISSN: 0168-1605, DOI: 10.1016/S0168-1605(03)00177-6 [retrieved on 2003-06-28]

## Description

### Technical Field

The present invention relates to a culture medium for detecting *Enterococcus,* and a method for detecting *Enterococcus* using the culture medium.

### Background Art

*Enterococcus* is a Gram-positive coccus, and is a series of bacteria that reside in an intestinal tract of mammals including human being. *Enterococcus* is deemed to be weak in pathogenicity, but is known to be responsible for postoperative endocarditis or nosocomial infection in several cases. In addition thereto, *Enterococcus* is also important as an indicator of fecal contamination, and therefore detection of *Enterococcus* is important in controlling a hygienic state of food or drinking water.

As a selective isolation culture medium of *Enterococcus,* a Kenner Fecal (KF) agar medium and an azide-citric acid (AC) broth medium are generally used (Non-patent literature No. 1). Such a culture medium contains 0.1% by weight or more of sodium azide as a selective agent of *Enterococcus.* However, a composition containing 0.1% by weight or more of sodium azide is specified to be strictly controlled as a poisonous substance. Therefore, in a commercially available culture medium, sodium azide is separately added thereto, or a powder culture medium already mixed therewith is required to be stored in a cabinet with a key (Non-patent literature No. 2).

In order to dissolve such complication on storage or distribution, a selective culture medium is required in which *Enterococcus* can be detected even if a content of sodium azide is less than 0.1% by weight. For example, a proposal has been made on a culture medium for detecting *Enterococcus* in which sodium azide in an amount less than 0.1 % by weight is combined with an aminoglycoside-based antibiotic and contained (Patent literature No. 1). Moreover, a proposal has been also made on a culture medium in which *Enterococcus* can be detected by containing crystal violet in a specific concentration without containing sodium azide (Patent literature No. 2).

Culture media used to isolate, count, characterize, and identify *Enterococcus* are disclosed in non-patent literature No. 3.

### Citation List

### Patent Literature

Patent literature No. 1: JP 2008-131897 A.
Patent literature No. 2: JP 2004-528856 A.

### [Non-Patent Literature]

Non-patent literature No. 1: Shin Saikinbaichigaku Koza (New Bacterial Culture Medium Science Course) - Ge(second) II - (second edition), under the editorship of Toshikazu Sakazaki, Author: Kazumitsu Tamura, Etsuro Yoshizaki, Kanji Miki, Kabushikigaisha Kindaishupan, issued Jan. 20, 1990.
Non-patent literature No. 2: Poisonous and Deleterious Substances Control Act, Appendix 1 No. 28, Cabinet Order No. 176, Final revision: Apr. 21, 2006.
Non-patent literature No. 3: Konrad J.D. et al., Int. J. Food Microbiol., 2003, 88, 147-164.

### Summary of Invention

### Technical Problem

However, from viewpoints of ease of handling a culture medium in preparation, storage thereof or the like, and detection accuracy, the culture medium for detecting *Enterococcus* has had room for further improvement.

In view of such a situation, the invention is contemplated for providing a culture medium for detecting *Enterococcus* in which the medium substantially contains no sodium azide, preparation thereof is simple, and *Enterococcus* can be selectively detected with high accuracy.

### Solution to Problem

The present inventors have diligently continued to conduct study in order to solve the problems as described above. As a result, the present inventors have found that *Enterococcus* can be selectively detected if an ionic surfactant such as bile acid, and polyvinyl alcohol are combined, and have completed the invention.

More specifically, the invention concerns the use of a culture medium as defined in claim 1 and a culture medium as defined in claim 6. The dependent claims are directed to some beneficial embodiments.

### Advantageous Effects of Invention

If a culture medium according to the invention is used, *Enterococcus* can be selectively detected with high accuracy by simple preparation. Moreover, the culture medium according to the invention has no necessity of incorporating sodium azide thereinto, and therefore has no complication on storage and distribution thereof, and therefore is useful.

### Description of Embodiments

A culture medium according to the invention contains (a) ionic surfactant and (b) polyvinyl alcohol.

In the invention, (a) ionic surfactant plays a role of suppressing growth of microorganisms other than *Enterococcus,* and a role of excluding colony thereof. In particular, Gram-positive bacteria have no cell envelope, and therefore a cell wall thereof is easily broken by surface active action, and growth thereof is preferably suppressed.

As the ionic surfactant, any of an anionic surfactant, a cationic surfactant and an amphoteric surfactant may be applied. Specific examples of the anionic surfactant include bile acid or salt thereof, sodium dodecyl sulfate (SDS) and N-lauroylsarcosine sodium salt. Specific examples of the cationic surfactant include benzalkonium chloride, benzethonium chloride, didecyldimethylammonium salt and hexadecyltrimethylammonium salt. Specific examples of the amphoteric surfactant include alkylpolyaminoethylglycine chloride, N-dodecyl-N,N-dimethyl-3-ammmonio-1-propanesulfonate, 3-[(3-cholamidopropyl)dimethylammonio]-1-propanesulfonate (CHAPS). Among the above surfactants, an anionic surfactant is further preferred, and bile acid or salt thereof, sodium dodecyl sulfate (SDS) or the like is still further preferred, and bile acid or salt thereof is particularly preferred. In addition, *Enterococcus* is known to have slightly higher resistance to bile acid in comparison with other Gram-positive bacteria.

Specific examples of the bile acid include Bile salts No.3, cholic acid, deoxycholic acid and taurodeoxycholic acid. Specific examples of the salt of bile acid include alkali metal salt such as sodium salt and potassium salt.

A content of the ionic surfactant in the culture medium according to the invention is preferably 1 to 10 g/L, and further preferably 1 to 6 g/L, as a concentration when the culture medium is used in a detection method as mentioned later (during growing bacteria, the same shall apply hereinafter). The microorganisms other than *Enterococcus,* particularly other Gram-positive bacteria can be preferably suppressed by adjusting the content to such a range.

In the invention, (b) polyvinyl alcohol plays a role as a gelling agent serving as a matrix for shaping the culture medium. Further, (b) polyvinyl alcohol plays a role of disabling growth suppressing action of (a) ionic surfactant *Enterococcus* only and providing the culture medium according to the invention with Enterococcus-specific selectivity.

As the polyvinyl alcohol, a material having a weight average molecular weight of preferably 5,000 to 200,000 and a degree of saponification of preferably 75 to 99%, and further preferably 85 to 90% is used.

A content of the polyvinyl alcohol in the culture medium according to the invention is preferably 140 to 300 g/L, and further preferably 160 to 220 g/L, as a concentration when the culture medium is used in the detection method as mentioned later. The culture medium can be easy handled and shaped, and selectivity of *Enterococcus* can be improved by adjusting the content to such a range.

Moreover, a mass ratio of the content of (a) ionic surfactant to (b) polyvinyl alcohol is adjusted to preferably 1 : 150 to 5 : 70, and further preferably 1 : 150 to 2 : 110. The selectivity of *Enterococcus* can be further improved by incorporating both materials thereinto at such a ratio.

The culture medium according to the invention further contains (c) suppressant on Gram-negative bacteria. Such an ingredient plays a role of suppresses the growth of Gram-negative bacteria and excluding the colony.

As the suppressant on Gram-negative bacteria, an antibiotic is preferred, and specific examples thereof include polymyxin B sulfate, colistin sulfate, aztreonam, carumonam and nalidixic acid, and polymyxin B sulfate and colistin sulfate are particularly preferred.

A content of the suppressant in the culture medium according to the invention is preferably 1 to 100 mg/L, and further preferably 10 to 30 mg/L as a concentration when the culture medium is used in the detection method as mentioned later.

The culture medium according to the invention further preferably contains (d) *β*-glucosidase substrate from which a colored chromogen compound can be released. Such an ingredient plays a role in which the ingredient is decomposed by *β*-glucosidase of *Enterococcus,* and the colored chromogen compound is released therefrom to form colored colony in *Enterococcus.*

Specific examples of such *β*-glucosidase substrate include 5-bromo-4-chloro-3-indoxyl-*β*-D-glucopyranoside, 5-bromo-3-chloro-4-indoxyl-*β*-D-glucopyranoside, 5-bromo-6-chloro-3-indoxyl-*β*-D-glucopyranoside, 3-indoxyl-*β* -D-glucopyranoside and esculin. Among the substrates, 5-bromo-4-chloro-3-indoxyl-*β*-D-glucopyranoside (X-GLUC) is further preferred in view of chromogenic properties and a small influence on growth of Enterococcus.
A content of the β-glucosidase substrate in the culture medium according to the invention is preferably 0.01 to 1.0 g/L, and further preferably 0.2 to 0.6 g/L as a concentration when the culture medium is used in the detection method as mentioned later.

The culture medium according to the invention is preferably substantially contains no sodium azide. Here, "substantially contains no sodium azide" means that a content of sodium azide is less than 0.1% by mass, preferably less than 0.01% by mass, and further preferably less than 0.001% by mass as a concentration when the culture medium is used in the detection method as mentioned later.

The culture medium according to the invention may arbitrarily contain, in addition to the ingredients, a nutritional ingredient, inorganic salts, a saccharide, a viscosity improver, and a pH adjuster.

As the nutritional ingredient, for example, peptone, an animal meat extract, a yeast extract or a fish meat extract is preferred.

Specific examples of the inorganic salts include inorganic acid metal salt such as sodium chloride and sodium thiosulfate, and organic acid metal salt such as sodium pyruvate, ferric ammonium citrate and sodium citrate.

Specific examples of the saccharide include lactose, sucrose, xylose, cellobiose and maltose.

Specific examples of the viscosity improver include a cellulose derivative such as methylcellulose, carboxymethylcellulose, hydroxyalkylcellulose; starch and a derivative thereof; a polysaccharide such as hyaluronic acid, guar gum and xanthan gum; an acrylic acid derivative such as polyacrylic acid, polyacrylate and an acrylic acid-vinyl alcohol copolymer; polyether such as polyethylene glycol and polypropylene glycol; and protein such as collagen.

Specific examples of the pH adjuster include sodium carbonate and sodium hydrogencarbonate.

Moreover, the culture medium according to the invention may contain an antibiotic on Gram-positive bacteria, and other antibacterial substances.

Specific examples of the antibiotic for Gram-positive bacteria include erythromycin and clindamycin.

Specific examples of other antibacterial substances include polylysine, protamine sulfate, glycine and sorbic acid. Moreover, in order to suppress growth of Eumycetes, an antibacterial compound such as amphotericin B may be incorporated thereinto.

Moreover, the culture medium according to the invention may contain an object drug in order to detect drug-resistant bacteria. For example, in order to detect vancomycin-resistant Enterococcus, vancomycin may be formulated.

Moreover, in the culture medium according to the invention, in view of growth of formed Enterococcus, pH during detection is preferably 6.0 to 8.0, and further preferably 6.5 to 7.5.

A form of the culture medium according to the invention is not particularly limited, and the culture medium can be prepared into a form of a solid culture medium which is cast into a petri dish or the like and solidified therein, and also into a sheet-shaped simple dry culture medium or the like.

Specific examples of the sheet-shaped simple dry culture medium include a sheetform culture medium having a configuration formed by laminating a layer in which a porous material is contained, a layer in which a gelling agent is contained, and a film layer and including the layers, as described in WO 97/24432 A, and the structure of the layer in which the gelling agent is contained only needs to be applied as the culture medium according to the invention.

The culture medium according to the invention can be preferably utilized in the method for detecting Enterococcus in the analyte. Such a method includes a step of inoculating the analyte into the culture medium, a step of culturing the microorganisms contained in the analyte and a step of detecting cultured colony of the microorganisms. Conditions in the culturing step are not particularly limited, but 24 to 48 hours at 35 ± 2°C are preferred.

The culture medium according to the invention suppresses the growth of Gram-positive bacteria other than Enterococcus, and does not inhibit the growth of Enterococcus. Therefore, according to the detection method according to the invention, Enterococcus can be easily selected and detected from the colony in which various Gram-positive bacteria can exist. According to a further preferred aspect, the culture medium according to the invention suppresses the growth of Gram-positive bacteria other than Enterococcus and Gram-negative bacteria, and does not inhibit the growth of Enterococcus. Therefore, according to the detection method according to the invention, Enterococcus can be easily selected and detected from the colony in which various bacteria can exist.

Specific examples of Enterococcus that can be selected and detected by the culture medium according to the invention preferably include E. faecalis, E. faecium, E. durans, E. gallinarum, E. casseliflavus, E. avium, E. hirae and E. raffinosus.

Specific examples of the analyte to be applied to the culture medium according to the invention include perishable food such as meat, fish and shellfish, a clinical analyte such as feces, drinking water, plain water, sea water and a wiping analyte in a cooking place and a hospital. However, a culture fluid obtained by preculturing the analytes in Tryptic Soy Broth or the like, and a culture fluid obtained by further culturing the culture fluid in an enrichment culture medium can also be used as the analyte.

### Examples

The invention will be described in greater detail by way of Examples. However, the invention is not limited by the Examples.

### Example 1 (not according to the present invention)

### (1) Preparation of culture medium

A total amount of a product obtained by adding each ingredient by 1 m² to 0.5 liter of purified water at a formulation shown in Table 1, and adjusting pH to 7.4 by anhydrous sodium carbonate, and warming and dissolving the resulting mixture at 95°C for 1 minute was uniformly applied onto a 20 *µ*m-thick polyester film having a dimension of 1 m × 1 m, and the resulting material was completely dried at 65°C. Then, a nylon meltblown nonwoven fabric (90 g/m²) was laminated thereon. Then, the resulting material was adhered onto a 100 *µ*m-thick polyester film having a dimension of 70 mm × 80 mm, and the resulting material was cut into 45 mm-square pieces to prepare sheet-shaped simple culture media, respectively. A detailed method for manufacturing the sheet-shaped simple culture medium was applied with reference to WO 97/24432 A.

Moreover, as controls, a commercially available Violet Red Bile Glucose agar medium (VRBG; made by Oxoid Limited) and a standard agar medium (SPC; made by Nissui Pharmaceutical Co., Ltd.) were also prepared.

### [Table 1]

**Table 1**

| (g/m²) | | | | |
|---|---|---|---|---|
| | Culture medium 1 | Culture medium 2 | Culture medium 3 | Culture medium 4 |
| Pepton | 2.5 | 2.5 | 2.5 | 2.5 |
| Meat peptone | 2.5 | 2.5 | 2.5 | 2.5 |
| Yeast extract | 1.5 | 1.5 | 1.5 | 1.5 |
| Bile salt No.3 | 0.75 | 0.75 | 0.75 | 1.5 |
| SDS | | | 0.3 | |
| Sodium chloride | 2.5 | 2.5 | 2.5 | 2.5 |
| Glucose | 5 | 5 | 5 | 5 |
| Neutral red | 0.015 | 0.015 | 0.015 | 0.015 |
| Crystal violet | 0.001 | 0.003 | 0.001 | 0.001 |
| Polyvinyl alcohol* | 109 | 109 | 109 | 109 |

| | | | | |
|---|---|---|---|---|
| * Kuraray Poval 217, weight average molecular weight: 5,000 to 200,000, degree of saponification: 87 to 89% (made by Kuraray Co., Ltd.). | | | | |

### (2) Sample provision of strain

A product obtained by culturing each bacterial strain shown in Table 2 in a Tryptic Soy agar medium for 24 hours was suspended into sterile physiological saline to be a concentration corresponding to McFarland nephelometry No. 1 (about 3.0 × 10⁸ CFU/ mL) by using a sterile cotton swab, and taken as a bacteria stock solution. Then, each bacteria stock solution was repeatedly subjected to dilution in 10-fold stages by sterile physiological saline into 10⁻⁷ CFU/mL, and then 1 mL of each bacteria solution having 10⁻⁷ or 10⁻² CFU/mL was provided as a sample for each culture medium. The sample was cultured at 35°C for 24 hours, and then the number of grown colony was counted. The results are shown in Table 2.

### [Table 2]

**Table 2**

| Sample bacteria and sample bacteria counts | | Culture medium 1 | Culture medium 2 | Culture medium 3 | Culture medium 4 | VRBG | SPC |
|---|---|---|---|---|---|---|---|
| (CFU/mL) | | | | | | | |
| *Escherichia coli* NBRC13500 | 10⁻⁷ | 117 | 140 | 120 | 106 | 144 | 140 |
| *Klebsiella oxytoca* JCM1665 | 10⁻⁷ | 113 | 113 | 107 | 101 | 91 | 136 |
| *Enterococcus faecalis* JCM7783 | 10⁻⁷ | 248 | 225 | 253 | 236 | -*¹ | 266 |
| *Enterococcus faecalis* JCM8726 | 10⁻⁷ | 182 | 193 | 178 | 172 | -*¹ | 186 |
| *Bacillus subtilis* NBRC3134 | 10⁻² | - | - | - | - | - | 33*² |
| *Staphylococcus aureus* NBRC100910 | 10⁻² | - | - | - | - | - | 34*² |

| | | | | | | | |
|---|---|---|---|---|---|---|---|
| (Colony counts) *1: 10⁻² CFU/mL sample, *2: 10⁻⁷ CFU/mL sample | | | | | | | |

In the culture medium 1, growth of B. subtilis and S. aureus in Gram-positive bacteria was suppressed, and Enterococcus (E. faecalis) achieved growth. In the VRBG agar medium, growth of Gram-positive bacteria including Enterococcus was generally suppressed. Growth of Enterococcus only was not suppressed in the Gram-positive bacteria also in the culture medium 2 containing crystal violet in an amount three times the amount of the culture medium 1, in the culture medium 3 to which SDS was added to the culture medium 1, and in the culture medium 4 containing bile salt in an amount two times the amount in the culture medium 1.

The culture medium according to the invention indicates that Enterococcus can be selectively detected even without containing sodium azide.

Moreover, the VRBG agar medium is different from the culture medium 1 only in using the agar as a gelling agent in place of polyvinyl alcohol, in which other formulations are almost the same. The suppressant exhibits no suppressing action on Enterococcus only by incorporating polyvinyl alcohol into the structure of the culture medium by simultaneously using the suppressant on Gram-positive bacteria, such as bile acid, SDS and crystal violet, which results were unpredictable from findings that have been so far obtained.

### Example 2

### (1) Preparation of culture medium

A sheet-shaped simple culture medium was prepared in a manner similar to Example 1 by adding each ingredient by 1 m² at a formulation shown in Table 3 to 0.5 liter of purified water, and adjusting pH to 7.4 by anhydrous sodium carbonate.

Moreover, as a control, a commercially available standard agar medium (SPC; made by Nissui Pharmaceutical Co., Ltd.) was also prepared.

### [Table 3]

**Table 3**

| (g/m²) | |
|---|---|
| | Culture medium 5 |
| Pepton | 2.5 |
| Meat peptone | 2.5 |
| Yeast extract | 1.5 |
| Bile salt No.3 | 1 |
| Sodium chloride | 2.5 |
| X-GLUC | 0.3 |
| Sodium pyruvate | 0.125 |
| Tween 80 | 1 |
| Polymyxin B sulfate | 0.01 |
| Polyvinyl alcohol* | 87 |

| | |
|---|---|
| * Kuraray Poval 217, weight average molecular weight: 5,000 to 200,000, degree of saponification: 87 to 89%, (made by Kuraray Co., Ltd.). | |

### (2) Sample provision of strain

With regard to each sample strain shown in Table 4, a bacteria stock solution was prepared, the resulting material was provided as a sample to a culture medium, and the bacteria was cultured in a manner similar to Example 1, and then the number of grown colony was counted, and color of the colony was observed. The results are shown in Table 4.

### [Table 4]

**Table 4**

| Sample bacteria and sample bacteria counts | | Culture medium 5 | SPC |
|---|---|---|---|
| (CFU/mL) | | | |
| *Enterococcus faecalis* JCM7783 | 10⁻⁷ | 87 (blue) | 92 |
| *Enterococcus faecalis* JCM8726 | 10⁻⁷ | 72 (blue) | 85 |
| *Bacillus subtilis* NBRC3134 | 10⁻² | - | 30^{*3} |
| *Staphylococcus aureus* NBRC100910 | 10⁻² | - | 28*³ |
| *Escherichia coli* NBRC13500 | 10⁻² | | 186*³ |
| *Pseudomonas aeruginosa* NBRC12689 | 10⁻² | | 63*³ |

| | | | |
|---|---|---|---|
| (Colony counts) *3: 10⁻⁷ CFU/mL sample | | | |

In the culture medium 5, growth of Gram-positive bacteria other than Enterococcus and Gram-negative bacteria was suppressed, and Enterococcus only achieved growth. Moreover, grown Enterococcus was easily detected as blue colony with high accuracy.

### Industrial Applicability

The invention provides a culture medium in which Enterococcus can be selectively detected with high accuracy by simple preparation. Moreover, the culture medium according to the invention has no necessity of incorporating sodium azide thereinto, and therefore has no complication on storage and distribution, and is industrially significantly useful.

## Claims

1. Use of a culture medium for detecting *Enterococcus,* wherein the culture medium comprises (a) ionic surfactant, (b) polyvinyl alcohol, and (c) suppressant on Gram-negative bacteria.

2. The use of claim 1, wherein the culture medium further comprises (d) β-glucosidase substrate from which a colored chromogen compound can be released.

3. The use of claim 1 or 2, wherein the (a) ionic surfactant is selected from the group of bile acid or salt thereof, sodium dodecyl sulfate (SDS) and N-lauroylsarcosine sodium salt.

4. The use of any one of claims 1 to 3, wherein the (b) polyvinyl alcohol has a weight average molecular weight of 5,000 to 200,000 and a degree of saponification of 75 to 99%.

5. The use of any one of claims 1 to 4, wherein the culture medium substantially contains no sodium azide.

6. A culture medium for detecting *Enterococcus,* comprising (a) ionic surfactant, (b) polyvinyl alcohol and (c) suppressant on Gram-negative bacteria.

7. The culture medium for detecting *Enterococcus* according to claim 6, further comprising (d) β-glucosidase substrate from which a colored chromogen compound can be released.

8. The culture medium for detecting *Enterococcus* according to claim 6 or 7, wherein the(a) ionic surfactant is selected from the group of bile acid or salt thereof, sodium dodecyl sulfate (SDS) and N-lauroylsarcosine sodium salt.

9. The culture medium for detecting *Enterococcus* according to any one of claims 6 to 8, wherein the (b) polyvinyl alcohol has a weight average molecular weight of 5,000 to 200,000 and a degree of saponification of 75 to 99%.

10. The culture medium for detecting *Enterococcus* according to any one of claims 6 to 9, substantially containing no sodium azide.

## Patentansprüche

1. Verwendung eines Kulturmediums zum Nachweis von Enterococcus, wobei das Kulturmedium (a) ein ionisches Tensid, (b) Polyvinylalkohol und (c) ein Unterdrückungsmittel auf gramnegative Bakterien umfasst.

2. Verwendung gemäß Anspruch 1, wobei das Kulturmedium ferner (d) β-Glucosidasesubstrat umfasst, von dem eine gefärbte Chromogenverbindung freigesetzt werden kann.

3. Verwendung gemäß Anspruch 1 oder 2, wobei das (a) ionische Tensid ausgewählt ist aus der Gruppe von Gallensäure oder Salz davon, Natriumdodecylsulfat (SDS) und N-Lauroylsarcosin-Natriumsalz.

4. Verwendung irgendeines der Ansprüche 1 bis 3, wobei der (b) Polyvinylalkohol ein gewichtsmittleres Molekulargewicht von 5.000 bis 200.000 und einen Verseifungsgrad von 75 bis 99% aufweist.

5. Verwendung irgendeinen der Ansprüche 1 bis 4, wobei das Kulturmedium im Wesentlichen kein Natriumazid enthält.

6. Kulturmedium zum Nachweis von Enterococcus, umfassend (a) ionisches Tensid, (b) Polyvinylalkohol und (c) Unterdrückungsmittel auf gramnegative Bakterien.

7. Kulturmedium zum Nachweis von Enterococcus gemäß Anspruch 6, ferner umfassend (d) β-Glucosidasesubstrat, von dem eine gefärbte Chromogenverbindung freigesetzt werden kann.

8. Kulturmedium zum Nachweis von Enterokokken gemäß Anspruch 6 oder 7, wobei das (a) ionische Tensid ausgewählt ist aus der Gruppe von Gallensäure oder Salz davon, Natriumdodecylsulfat (SDS) und N-Lauroylsarcosin-Natriumsalz.

9. Kulturmedium zum Nachweis von Enterococcus gemäß irgendeinem der Ansprüche 6 bis 8, wobei der (b) Polyvinylalkohol ein gewichtsmittleres Molekulargewicht von 5.000 bis 200.000 und einen Verseifungsgrad von 75 bis 99% aufweist.

10. Kulturmedium zum Nachweis von Enterococcus gemäß irgendeinem der Ansprüche 6 bis 9, das im Wesentlichen kein Natriumazid enthält.

## Revendications

1. Utilisation d'un milieu de culture pour la détection *d'Enterococcus,* dans laquelle le milieu de culture comprend (a) un tensioactif ionique, (b) de l'alcool polyvinylique, et (c) un suppresseur sur les bactéries Gram négatif.

2. Utilisation selon la revendication 1, dans laquelle le milieu de culture comprend en outre (d) un substrat de β-glucosidase à partir duquel un composé chromogène coloré peut être libéré.

3. Utilisation selon la revendication 1 ou 2, dans laquelle le (a) tensioactif ionique est sélectionné dans le groupe de l'acide biliaire ou d'un sel de celui-ci, du dodécyl sulfate de sodium (SDS) et d'un sel sodique de N-lauroylsarcosine.

4. Utilisation selon l'une quelconque des revendications 1 à 3, dans laquelle le (b) alcool polyvinylique a une masse moléculaire moyenne en poids de 5 000 à 200 000 et un degré de saponification de 75 à 99 %.

5. Utilisation selon l'une quelconque des revendications 1 à 4, dans laquelle le milieu de culture ne contient sensiblement pas d'azoture de sodium.

6. Milieu de culture pour la détection d'*Enterococcus*, comprenant (a) un tensioactif ionique, (b) de l'alcool polyvinylique et (c) un suppresseur sur les bactéries Gram négatif.

7. Milieu de culture pour la détection *d'Enterococcus* selon la revendication 6, comprenant en outre (d) un substrat de β-glucosidase à partir duquel un composé chromogène coloré peut être libéré.

8. Milieu de culture pour la détection *d'Enterococcus* selon la revendication 6 ou 7, dans lequel le (a) surfactant ionique est sélectionné dans le groupe de l'acide biliaire ou d'un sel de celui-ci, du dodécyl sulfate de sodium (SDS) et d'un sel sodique de N-lauroylsarcosine.

9. Milieu de culture pour la détection *d'Enterococcus* selon l'une quelconque des revendications 6 à 8, dans lequel le (b) alcool polyvinylique a une masse moléculaire moyenne en poids de 5 000 à 200 000 et un degré de saponification de 75 à 99 %.

10. Milieu de culture pour la détection d'*Enterococcus* selon l'une quelconque des revendications 6 à 9, ne contenant sensiblement pas d'azoture de sodium.
